(19) Europäisches Patentamt
European Patent Office
Office européen des brevets

(11) Veröffentlichungsnummer: **0 253 214 B1**

# (12) EUROPÄISCHE PATENTSCHRIFT

(45) Veröffentlichungstag der Patentschrift: **28.03.90**

(51) Int. Cl.⁴: **C07C 53/50**, C07C 51/60

(21) Anmeldenummer: **87109505.5**

(22) Anmeldetag: **02.07.87**

## (54) Verfahren zur Herstellung von Chlorcarbonsäurechloriden.

(30) Priorität: **18.07.86 DE 3624258**

(43) Veröffentlichungstag der Anmeldung: **20.01.88 Patentblatt 88/3**

(45) Bekanntmachung des Hinweises auf die Patenterteilung: **28.03.90 Patentblatt 90/13**

(84) Benannte Vertragsstaaten: **BE CH DE FR GB IT LI**

(56) Entgegenhaltungen: **DE-A-1 568 855**

(73) Patentinhaber: **BASF Aktiengesellschaft, Carl-Bosch-Strasse 38, D-6700 Ludwigshafen(DE)**

(72) Erfinder: **Decker, Martin,Dr., Maria-Stuart-Strasse 21, D-6700 Ludwigshafen(DE)**
Erfinder: **Neumayr, Franz, Dr., Im Eiertal 8, D-6719 Weisenheim(DE)**

**EP 0 253 214 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Diese Erfindung betrifft ein Verfahren zur Herstellung von Chlorcarbonsäurechloriden durch Umsetzung von Lactonen mit Phosgen in Gegenwart von quartären Ammoniumsalzen.

Aus der FR-PS 1 080 261 ist bekannt, daß man 4-Chlorbuttersäurechlorid dadurch herstellen kann, daß man 4-Butyrolacton bei ungefähr 120°C mit Phosgen umsetzt, wobei man als Katalysator Pyridin verwendet. Da es nicht möglich war, das in Beispiel 2 dieser Patentschrift angegebene Ergebnis zu reproduzieren, und Chlorcarbonsäurechloride in den letzten Jahren zunehmende Bedeutung als Vorprodukte für die Herstellung von Arzneimitteln und Pflanzenschutzmitteln erlangt haben, war nach einem Verfahren zu suchen, das es ermöglicht, Chlorcarbonsäurechloride auf technisch wirtschaftliche Weise und mit guten Ausbeuten herzustellen.

Es wurde nun gefunden, daß man Chlorcarbonsäurechloride der Formel

$$R-\underset{\underset{\displaystyle Cl}{|}}{C}H-(CH_2)_n-COCl \qquad I,$$

in der R H oder einen Alkylrest und n eine ganze Zahl von 2 bis 5 bedeuten, durch Umsetzung von Lactonen der Formel

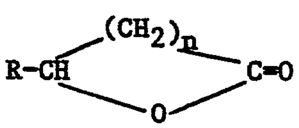 II .

mit Phosgen bei höherer Temperatur besonders vorteilhaft herstellen kann, wenn man die Umsetzung in Gegenwart von quartären Ammoniumsalzen durchführt.

In den Ausgangsstoffen der Formel II steht R für H oder einen Alkylrest mit z.B. 1 bis 3 C-Atomen. Als geeignete Lactone der Formel II kommen z.B. im Betracht: 4-Butyrolacton, 5-Methyl-4-butyrolacton, 5-Valerolacton und 6-Caprolacton.

Als quartäre Ammoniumsalze verwendet man z.B. Trimethylbenzylammoniumchlorid, N,N-Dimethylpiperidiniumchlorid, Dimethylmorpholiniumchlorid. Man verwendet sie in Mengen von 0,5 bis 5 Mol %, bezogen auf das Lacton. Diese Verbindungen werden unter den Reaktionsbedingungen von Phosgen nicht angegriffen und ihre Wirksamkeit bleibt demgemäß sehr lange erhalten.

Das erfindungsgemäße Verfahren wird z.B. bei Temperaturen von 60 bis 200°C, vorzugsweise bei 90 bis 180°C durchgeführt. Zweckmäßigerweise verfährt man so, daß man das Lacton, gegebenenfalls zusammen mit einem inerten Lösungsmittel, und das quartäre Ammoniumsalz vorlegt, das Gemisch auf Reaktionstemperatur erwärmt und dann Phosgen einleitet. Phosgen wird in stöchiometrischen oder darüberliegenden Mengen, z.B. bis zur 3-fachen stöchiometrischen Menge eingesetzt, wobei man den Phosgenüberschuß vorteilhaft durch Kühlung der Abgase auf mindestens -40, vorzugsweise mindestens -70°C auskondensiert und in das Reaktionsgefäß zurückleitet. Als inerte Lösungsmittel sind solche Lösungsmittel geeignet, die unter den Reaktionsbedingungen nicht mit den Ausgangsstoffen und den Reaktionsprodukten reagieren, das sind z.B. Cumol oder andere hochsiedende Kohlenwasserstoffe, sowie aromatische Chlorverbindungen, wie Dichlorbenzol. Im allgemeinen ist ein Lösungsmittel jedoch nicht erforderlich. Das Verfahren wird zweckmäßig bei Normaldruck ausgeübt oder bei Drücken, die nur wenig vom Normaldruck abweichen. Es kann diskontinuierlich oder kontinuierlich durchgeführt werden.

Nach einer besonders vorteilhaften Ausführungsform des erfindungsgemäßen Verfahrens leitet man gleichzeitig mit dem Phosgen bis zu 100 Mol% bezogen auf das Lacton, an Chlorwasserstoff oder an solchen Verbindungen in das Reaktionsgefäß ein, die mit Phosgen Chlorwasserstoff bilden, wie z.B. Wasser. Vorzugsweise gibt man 5 bis 100, insbesondere 20 bis 40 Mol.% Chlorwasserstoff oder Chlorwasserstoff bildende Stoffe, bezogen auf das Lacton zu. Überraschenderweise hat sich gezeigt, daß durch diese Zusätze die Umsetzung des Lactons zu den Chlorcarbonsäurechloriden stark beschleunigt wird. Schon kleine Mengen dieser Zusätze zeigen einen deutlichen Effekt. Größere Mengen an Chlorwasserstoff sind aus wirtschaftlichen Gründen und wegen des Strippeffektes auf das im Reaktionsgemisch gelöste Phosgen nicht zu empfehlen.

Nach dem Verfahren der Erfindung werden die Chlorcarbonsäurechloride glatt und mit hoher Ausbeute erhalten.

Die in den Beispielen genannten Teile sind Gewichtsteile.

<u>Beispiel 1</u>

In einem Rührreaktor mit der Zuführmöglichkeit von Chlorwasserstoff und Phosgen werden 1 032 Teile 4-Butyrolacton und 54 Teile N,N-Dimethylpiperidiniumchlorid vorgelegt und die Innentemperatur durch ein Heizmedium auf 130 bis 135°C gebracht. Bei dieser Temperatur werden im Laufe von 10 Stunden 1 200 Teile Phosgen und 150 Teile Chlorwasserstoff einge leitet. Aus dem Abgas wird das Phosgen durch Kühlung auf -70°C auskondensiert und in die Reaktionszone zurückgeführt. Nach beendeter Zugabe wird das Reaktionsgemisch zur Vervollständigung der Reaktion noch 2 Stunden bei dieser Temperatur gehalten. Danach wird bei ca. 100 mbar das 4-Chlorbuttersäurechlorid über eine Kolonne abdestilliert. Zum Rückstand, der aus monomerem und oligomerem 4-Butyrolacton und Katalysator beshteht, werden erneut 1 032 Teile 4-Butyrolactor gegeben und wie oben beschrieben mit Phosgen und Chlorwasserstoff umgesetzt. Auf diese Weise werden insgesamt 5 160 Teile Butyrolacton eingesetzt. Nach destillativer Aufarbeitung erhält man 8 012 Teile 4-Chlorbuttersäurechlorid, entsprechend einer Ausbeute von 94,7 Mol-%.

Beispiel 2

In einem 1 l-Rührreakton, der mit Thermometer, Einleitungsrohren für Phosgen und Chlorwasserstoff sowie mit 2 Tiefkühlern ausgerüstet ist, die auf Temperaturen von -20 bzw. -70°C eingestellt sind, werden 430 Teile 4-Butyrolacton und 47 Teile Trimethylbenzylammoniumchlorid vorgelegt. Die Mischung wird auf 130°C erwärmt. Im Zeitraum von 5,5 h werden 560 Teile Phosgen und 120 Teile Chlorwasserstoff eingeleitet. Danach wird so lange Stickstoff eingeleitet, bis die Reaktionsmischung kein Phosgen mehr enthält. Durch destillative Aufarbeitung werden 620 g 4-Chlorbuttersäurechlorid und 30,5 g Butyrolacton erhalten. Der Destillationsrückstand von 75 g, welcher aus Katalysator und Chlorbutyroyloxibuttersäurechlorid besteht, kann für weitere Ansätze wiederverwendet werden. Die Ausbeute an 4-Chlorbuttersäurechlorid, bezogen auf eingesetztes Butyrolacton, beträgt 88 Mol-%.

Beispiel 3

Entsprechend Beispiel 1 werden 100 Teile 4-Valerolacton in Gegenwart von 4,5 Teilen N,N-Dimethylpiperidiniumchlorid bei 140 - 155°C im Laufe von 10 Stunden mit 80 Teilen Phosgen und 100 Teilen gasförmigem Chlorwasserstoff behandelt. Durch Aufarbeitung und Destillation analog Beispiel 1 werden 60,2 Mol.% 4-Chlorpentansäurechlorid, bezogen auf umgesetztes 4-Valerolacton erhalten. Der Umsatz beträgt 54 %. Durch Wiedereinsatz des oligomeren Destillationsrückstandes kann die Ausbeute noch erhöht werden.

Beispiel 4

Entsprechend Beispiel 1 werden 100 Teile 5-Valerolacton in Gegenwart von 4,5 Teilen N,N-Dimethylpiperidiniumchlorid bei 170 - 175°C im Laufe von 2,5 Stunden mit 100 Teilen Phosgen umgesetzt. Der Umsatz beträgt 93 %. Durch Aufarbeitung und Destillation analog Beispiel 1 werden 76,3 Mol.% 5-Chlorpentansäurechlorid, bezogen auf umgesetztes 5-Valerolacton, erhalten. Durch den Wiedereinsatz des oligomeren Destillationsrückstandes bei Folgeansätzen wird die Ausbeute an 5-Chlorpentansäurechlorid noch erhöht.

Beispiel

Entsprechend Beispiel 1 werden 114 Teile 6-Caprolacton in Gegenwart von 4,5 Teilen N,N-Dimethylpiperidiniumchlorid bei 160 - 170°C im Laufe von 4,75 Stunden mit 100 Teilen Phosgen umgesetzt. Der Umsatz beträgt 78,9 %. Durch Aufarbeitung und Destillation analog Beispiel 1 werden 81,7 Mol-% 6-Chlorhexansäurechlorid bezogen auf umgesetztes 6-Caprolacton erhalten. Durch den Wiedereinsatz des oligomeren Destillationsrückstandes wird die Ausbeute an 6-Chlorhexansäurechlorid noch erhöht.

Beispiel 6

In dem in Beispiel 1 beschriebenen Rührreaktor werden 456 Teile 6-Caprolacton und 9 Teile N,N-Dimethylpiperidiniumchlorid vorgelegt. Bei 170 bis 175°C werden 380 Teile Phosgen und 70 Teile Chlorwasserstoff im Zeitraum von 7 Stunden eingeleitet. Danach wird die Reaktionsmischung noch 1 Stunde bei dieser Temperatur gehalten. Noch vorhandenes Phosgen und Chlorwasserstoff werden mit Stickstoff ausgeblasen. Danach wird das Reaktionsgemisch durch Vakuumdestillation aufgearbeitet. Es werden 424 Teile 6-Chlorhexansäurechlorid und 86 Teile Caprolacton erhalten. Der Umsatz beträgt 81 %, die Ausbeute, bezogen auf eingesetztes Lacton 77,3 %.

Beispiel 7

Analog Beispiel 6 werden 200 Teile 5-Valerolacton und 9 Teile Dimethylpiperidiniumchlorid auf 175 bis 180°C erhitzt und bei dieser Temperatur mit 230 Teilen Phosgen und 70 Teilen Chlorwasserstoff umgesetzt. Die Reaktionsdauer beträgt 3,75 Stunden. Danach werden gelöstes Phosgen und Chlorwasserstoff mit Stickstoff ausgeblasen und das Rohprodukt im Vakuum destilliert.

Es werden 241 Teile 5-Chlorpentansäurechlorid mit einer Reinheit von 98,1 % erhalten. Im Destillationsrückstand von 52 Teilen sind der Katalysator und nicht umgesetztes 5-Valerolacton enthalten, die bei Folgeansätzen wieder eingesetzt werden. Die Ausbeute bezogen auf eingesetztes Lacton, beträgt 76,2 %.

**Patentansprüche**

1. Verfahren zur Herstellung von Chlorcarbonsäurechloriden der Formel

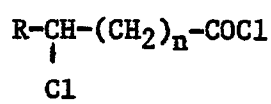

I,

in der R H oder einen Alkylrest und n eine ganze Zahl von 2 bis 4 bedeuten, durch Umsetzung von Lactonen der Formel

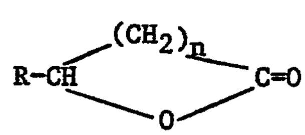

II

mit Phosgen bei höherer Temperatur, dadurch gekennzeichnet, daß man die Umsetzung in Gegenwart von quartären Ammoniumsalzen durchführt.

2. Verfahren nach Anspruch 1, dadurch gekennzeichnet, daß man das Lacton und das quartäre Ammoniumsalz in dem Reaktionsgefäß vorlegt, und das Phosgen sowie bis zu 100 Mol-%, bezogen auf das Lacton, an Chlorwasserstoff oder einer Verbindung, die mit Phosgen Chlorwasserstoff bildet, in das Reaktionsgefäß einleitet.

3. Verfahren nach Anspruch 1, dadurch gekennzeichnet, daß man die Umsetzung bei 60 bis 200°C durchführt.

4. Verfahren nach Anspruch 1, dadurch gekennzeichnet, daß man die Umsetzung bei 90 bis 180°C durchführt.

5. Verfahren nach Anspruch 1, dadurch gekennzeichnet, daß man 5 bis 100 Mol-%, bezogen auf das Lacton, an Chlorwasserstoff oder der Chlorwasserstoff bildenden Verbindung einsetzt.

6. Verfahren nach Anspruch 1, dadurch gekennzeichnet, daß man als quartäres Ammoniumsalz N,N-Dimethylpiperiniumchlorid verwendet.

**Claims**

1. A process for the preparation of a chlorocarboxylic acid chloride of the formula

$R-CH-(CH_2)_n-COCl$ I
Cl

where R is H or alkyl and n is an integer from 2 to 4, by reacting a lactone of the formula

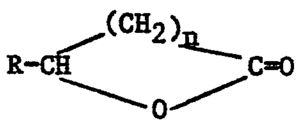
II

with phosgene at elevated temperatures, wherein the reaction is carried out in the presence of a quaternary ammonium salt.

2. A process as claimed in claim 1, wherein the lactone and the quaternary ammonium salt are initially taken in the reaction vessel, and the phosgene and up to 100 mol %, based on the lactone, of hydrogen chloride or of a compound which forms hydrogen chloride with phosgene is passed into the reaction vessel.

3. A process as claimed in claim 1, wherein the reaction is carried out at from 60 to 200°C.

4. A process as claimed in claim 1, wherein the reaction is carried out at from 90 to 180°C.

5. A process as claimed in claim 1, wherein from 5 to 100 mol %, based on the lactone, of hydrogen chloride or of the compound which forms hydrogen chloride is used.

6. A process as claimed in claim 1, wherein the quaternary ammonium salt used is N,N-dimethylpiperidinium chloride.

**Revendications**

1. Procédé de préparation de chlorures d'acides chlorocarboxyliques de formule

$R-CH-(CH_2)_n-COCl$ I,
Cl

dans laquelle R représente un atome d'hydrogène ou un reste alkyle et n un entier de 2 à 4, par réaction de lactones de formule

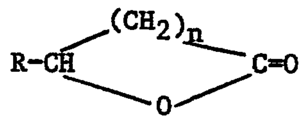
II

avec le phosgène à températures élevées, caractérisé en ce qu'on effectue la réaction en présence de sels d'ammonium quaternaires.

2. Procédé selon la revendication 1, caractérisé en ce qu'on introduit au préalable la lactone et le sel d'ammonium quaternaire dans le récipient de réaction et on fait passer dans le récipient de réaction le phosgène ainsi que jusqu'à 100% en moles, par rapport à la lactone, d'acide chlorhydrique ou d'un composé qui forme de l'acide chlorhydrique avec le phosgène.

3. Procédé selon la revendication 1, caractérisé en ce qu'on effectue la réaction à 60–200°C.

4. Procédé selon la revendication 1, caractérisé en ce qu'on effectue la réaction à 90–180°C.

5. Procédé selon la revendication 1, caractérisé en ce qu'on introduit 5 à 100% en moles, par rapport à la lactone, d'acide chlorhydrique ou de composé produisant de l'acide chlorhydrique.

6. Procédé selon la revendication 1, caractérisé en ce qu'on utilise comme sel d'ammonium quaternaire du chlorure de N,N-diméthylpipéridinium.